# EUROPEAN PATENT SPECIFICATION

(11) **EP 3 328 335 B1**
(45) Date of publication and mention of the grant of the patent: **17.02.2021**
(21) Application number: 16830920.1
(22) Date of filing: 03.06.2016
(51) Int. Cl.: A61F 9/06, A41D 13/11, A41D 13/12, A42B 3/22

(54) **LIGHT FRAME FOR VISOR**
LICHTRAHMEN FÜR VISIER
CADRE LUMINEUX POUR VISIÈRE

(30) Priority: 28.07.2015 SE 1551045
(43) Date of publication of application: 06.06.2018
(73) Proprietor: LENSK, 168 62 Bromma (SE)
(72) Inventor: DAUTOV, Azamat, 168 62 BROMMA (SE)
(74) Representative: Noréns Patentbyrå AB
(86) International application number: PCT/SE2016/050528
(87) International publication number: WO 2017/018919

(56) References cited:
- DE-A1-102012 000 793
- DE-A1-102012 000 793
- US-A1- 2001 021 108
- US-A1- 2002 089 844
- US-A1- 2002 089 844
- US-A1- 2007 261 153
- US-A1- 2011 013 135
- US-B1- 6 824 265
- US-B1- 6 824 265
- US-B1- 7 566 139

## Description

The present invention relates to a light frame for use with a visor, in particular to such a light frame adapted for use with or on a visor for illuminating a work area for a user of the visor.

In many activities, transparent visors are used, often in order to protect the eyes of a user during different types of work. Examples include work during surgical procedures, work in different types of cleanrooms and welding work.

In the latter example, such visors often automatically dimmed, so that the visor translucency is decreases as a result of a sudden increase in the inflow of light. This in itself provides improved lighting ergonomics during welding, as the risk of dazzling is reduced, however such automatically dimmed visors are not completely transparent, even in poor lighting. In many cases, the welding work has to be performed under such poor lighting, such as in oil pits under cars. In addition, certain work must be performed in narrow spaces. The same is also true in many cases outside the field of welding.

To solve these problems, a separate light source such as a flashlight or a head lamp may be used. However, this is often not practical, because there may be no place to hold and/or to secure such a lamp, and/or since such a lamp in many cases does not provide adequate illumination of the working area.

Documents DE 102012000793 A1, CN 203 001 229 U, US 6 340 234 B1, DE 202013101085 U1, US 7 161116 B2, US 8 721 103 B2, US 7 934 846 B1, US 20050190549 A1 and US 20110107491 A1 disclose prior art illumination solutions. These known solutions suffer, to varying degrees, from the problems described above, in particular the problem of providing adequate ergonomics, and providing lighting in dark, particularly narrow spaces, especially when using an existing visor.

US 6824265 B1 describes a pair of glasses with built-in illumination light sources. Said light sources are arranged on either side of the glasses, and directed forward at different vertical angles.

US 7566139 B1 discloses an illumination device for use on magnifying headgear that provides for selective magnification of objects. The device comprises LEDs for uniform illumination of a viewed object.

The present invention solves the above described problems.

Hence, the invention relates to a light frame for use with a visor, which light frame comprises a rigid frame part arranged to be mounted around the periphery of a sight glass of the visor using fastening means of the light frame arranged for attaching the light frame to the visor, which frame part comprises at least three directed light sources, with respective light cones, arranged to illuminate the area in front of sight glass during use, which light frame further comprises a voltage source arranged to power the light sources, and is characterised in that said light sources comprise at least two fixedly directed primary light sources, whose light cones are directed towards the same point arranged between 20 and 80 cm in front of the sight glass during use, and in that said light sources further comprise at least one fixedly directed secondary light source, whose light cone during use is not directed towards the said point, in that different pairs of respective primary light sources are mounted on the frame part so that they are disposed on different sides of the sight glass during use, and in that at least two such pairs of primary light sources are arranged to, during use, be directed towards different common points in front of the sight glass.

Furthermore the invention relates to a welding helmet, comprising a sight glass and a light frame as described above, whereby the light frame is arranged around the periphery of the sight glass.

In the following, the invention will be described in detail, with reference to the accompanying drawings, in which
Figure 1 is a perspective view of a light frame according to the present invention;
Figure 2a shows a light frame according to the invention mounted on a welding helmet as seen from the side;
Figure 2b shows the same light frame as in Figure 2a, as seen from above; and
Figure 3 is a perspective view of a light frame according to the invention, comprising a head-band, for mounting on a visor.

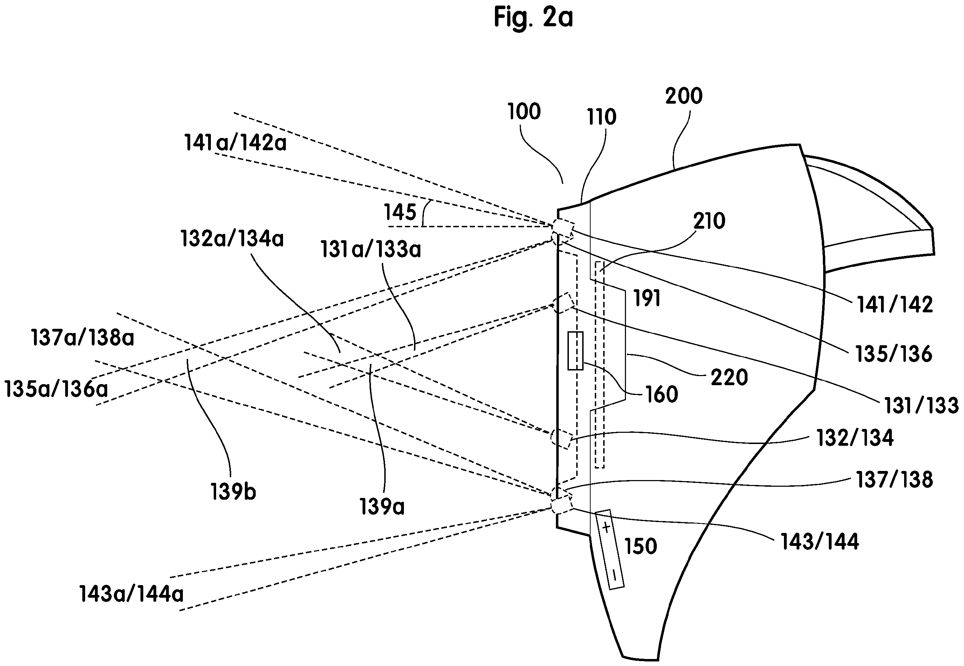

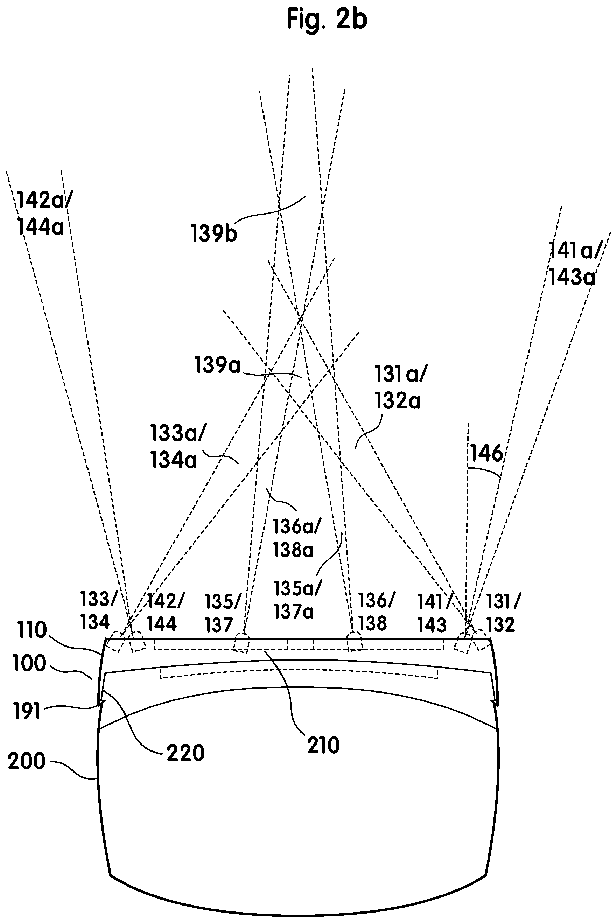

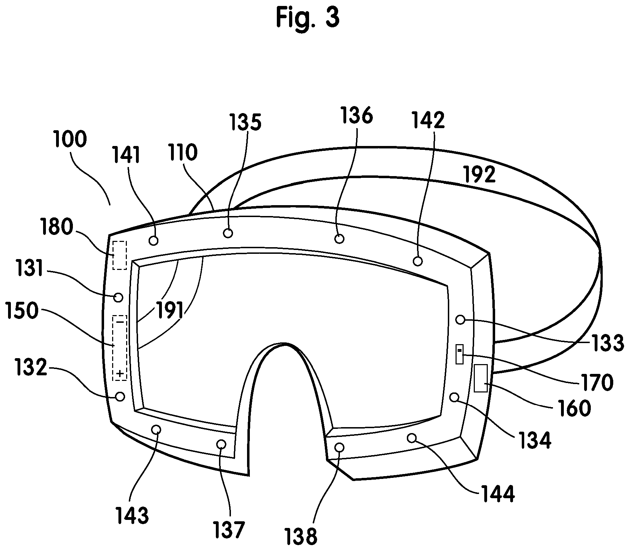

All Figures share reference numerals for same or corresponding parts. It will be appreciated that the visor in Figures 2a and 2b is comprised in the welding helmet. In Figure 3, a visor, onto which the light frame is adapted to be fastened, is not illustrated. In this latter case, a visor can be of any type, such as a visor for use in for example surgical work.

The present invention thus relates to a light frame 100 for use with a visor (see Figures 2a and 2b). Such a visor may be a welding helmet or any other type of equipment that constitutes or comprises a visor. That the light frame 100 is "for use" with a visor means that it is specifically designed for such use, preferably specifically adapted to be used with a defined type of visor, which type of visor can be different for different practical applications of the present invention. Such adaptation comprises both a shape-wise adaptation for being able to be brought into the below-described mounted state of the visor, but may also comprise the light frame 100 being provided with any of the types of fastening means 191, 192 described below for attaching the light frame 100 to the visor.

The light frame 100 comprises a rigid frame part 110, arranged for, during use, being mounted about the periphery of a sight glass 210 of the visor. This represents herein a "mounted state" of the light frame with respect to the visor.

Furthermore, the frame part 110 comprises at least three directed light sources 131-138; 141-144, with respective light cones 131a-138a; 141a-144a. Said cones are arranged to illuminate the area in front of the sight glass 210 during use, when the light frame 100 is in said mounted state.

Moreover, the light frame 100 comprises a voltage source 150, arranged to power the light sources 131-138; 141-144.

An essential aspect of the present invention is that different light sources 131-138, 141-144 are arranged to provide respective light cones 131a-138a; 141a-144a with at least two different principal angles.

According to the invention, said light sources 131-138; 141-144 comprise at least two fixedly directed primary light sources 131-138, the light cones 131a-138a of which are directed towards one and the same common point 139a, 139b arranged between 20 and 80 cm, preferably between 40 and 60 cm, preferably about 50 cm, in front of the sight glass 210 during use, when the light frame 100 is in said mounted state. Preferably, said common point 139a, 139b is arranged substantially straight in front of the sight glass 210 during such use, such as perpendicularly to the sight glass 210.

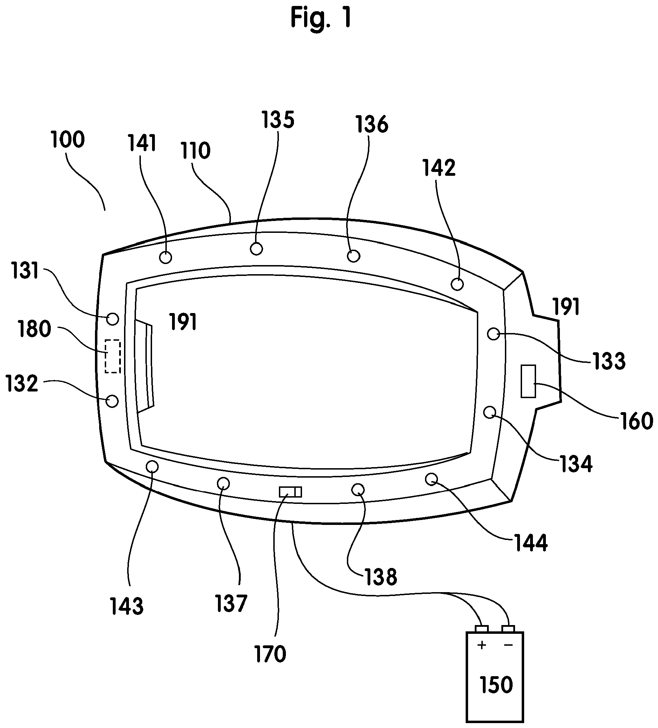

It is understood that in the exemplary embodiment illustrated in Figure 1, the light frame 100 comprises not only two primary light sources 131-138, but at least four, and these four primary light sources 131, 133, 135, 136 are also arranged to direct their respective light cones 131a, 133a, 135a, 136a in pairs towards two different points 139a, 139b, each of which therefore constituting a respective "common point" for a respective such pair of primary light sources 131, 133; and 135, 136, according to the invention. It is preferred that said multiple common points 139a, 139b are arranged at different distances from the sight glass 210 during said use, preferably substantially in line with each other as seen from the sight glass 210. It is appreciated that the figures illustrate eight primary light sources 131-138, arranged to direct their respective light cones 131a-138a towards two common points 139a, 139b four by four.

In addition thereto, said light sources 131-138; 141-144 according to the invention comprise at least one fixedly directed secondary light source 141-144, whose light cone, during use when light frame 100 is in said mounted state, is not directed towards the common point 139a, 139b. In the case in which several common points 139a, 139b are used, it is preferred that none of the secondary light sources 141-144 is directed towards any one of these common points 139a, 139b. In an analogous manner as for the primary light sources 131-138, it is understood that in the exemplary embodiment illustrated in Figure 1, the number of secondary light sources 141-144 is four, but it is sufficient with one such secondary light source 141-144 to achieve the benefits of the invention to at least some extent.

It will be appreciated that the light cones 131a-138a; 141a-144a have a certain extension perpendicularly to their respective main longitudinal direction, and what is said herein regarding "common points," to be "directed towards" and so on should be construed to refer to the areas subjected to the main light intensity of the light cones 131a- 138a; 141a-144a. Thus, a "common point" may have a certain lateral extension, which extension is determined by the surface across which there is a lateral overlap occurring between two or more primary light cones 131a-138a.

Such a light frame offers very good lighting ergonomics when working in the dark, especially in cramped spaces. The primary light sources 131-138 provide good lighting, free from annoying shadows on the portion of the work area that is currently being considered for work, and with the opportunity to avoid that protruding geometrical details obscure the lighting. The one or more secondary light sources 141-144 provide both general lighting and peripheral illumination for general orientation of the user and the lighting of areas outside of the immediate work area, which is useful for general orientation, but also for example when users reach out for tools, etc., without for that reason desiring a reduction of the illumination of the immediate work area, or when it is not possible to turn the visor due to a lack of space.

Said at least one secondary light sources 141-144 are preferably directed substantially perpendicularly to the sight glass 210, such as completely perpendicularly or slightly divergently directed in relation to said common point.

According to the present invention, the light frame 100 comprises respective primary light sources 131, 133; 132, 134; 135, 137; 136, 138, mounted on the frame part 110 so that they are disposed on either side of the sight glass 210 during use, for example separated in the vertical or horizontal direction on either side of the sight glass 210. This provides good suppression of disturbing shadows on the work area. Similarly, it is preferred that the light frame 100 further comprises respective secondary light sources 141, 143; 142, 144; but also 141, 142; 143, 144, mounted on frame part 110 so that they are disposed on either side of the sight glass 210 during use, such as separated in the vertical or the horizontal direction on either side of the sight glass 210. This provides good general illumination. In addition, a combination of such primary and secondary light sources, separated on the frame part 110, achieves very good overall lighting ergonomics, with good shadow suppression, over a very wide area comprising the work area.

As for the secondary light sources 141-144, it is preferred that they, in addition to being disposed in a separated manner as described above, are also arranged in pairs at a substantially horizontal distance from each other on the visor during use in said mounted state, and that one or more of such pairs of secondary light sources 141, 143; 142, 144; 141, 142; 143, 144 are divergently directed with a relative angle between them of more than 0°, preferably at the most 10°. In the figures, angles 145 and 146 are illustrated as the angle between a respective light cone 141a-144a and a line perpendicular to the main plane of the light frame 100. It is understood that this angle of at the most 10° is thus double the angle illustrated as 145, 146, in the preferred case in which the divergence between the secondary light source pairs is symmetric around such a perpendicular line.

According to a particularly preferred embodiment, the sight glass 210 has a substantially angular shape with outer corners, wherein respective secondary light sources 141, 142, 143, 144 are mounted on the frame part 110 so that they, during use and in said mounted state, are disposed at different respective outer corners of the sight glass 210, at three or more locations, that is, at three different outer corners. Preferably, respective secondary light sources 141-144 are then arranged at different respective outer corners in at least four locations. In the preferred case in which the sight glass 210 is substantially rectangular, it is preferred that respective secondary light sources 141-144 are arranged at each of the four outer corners of the sight glass 210. It is this exemplary embodiment which is illustrated in the Figures.

According to a preferred embodiment, different pairs 131, 133 and 132, 134 of primary light sources are mounted on the frame part 110 such that they are disposed on either side of the sight glass 210 during use and in the mounted state, preferably on both sides in the horizontal plane. In this case, each such pair 131, 133; 132, 134 of primary light sources is preferably arranged to, during use and in the mounted state, be directed towards the same common point 139a in front of the sight glass 210. Similarly, other respective pairs 135, 137; 136, 138 of primary light sources may be arranged on either vertical sides of the sight glass 210 during use and in the mounted state, and then be directed towards another common point 139b. In this and in other cases, such various common points 139a, 139b may be arranged at different respective distances from the sight glass for different such pairs of primary light sources. In the Figures, the point 139a is shown to be closer than point 139b from the sight glass 210. It is preferred that the common point 139a arranged closest to the sight glass 210 is arranged at the above said distance of between 20 and 80 cm, preferably between 40 and 60 cm, preferably about 50 cm, from the sight glass 210. Remaining common points 239b are then preferably disposed at greater distances from the sight glass 210, such as between 20 and 40 cm further away from the sight glass 210 than the closest common point 139a.

It is preferred that the primary light sources 131-134; 135-138 being directed towards a single common point 139a and 139b are also directed towards each other with a relative angle between their respective light cones 131a-134a; 135a-138a of between 5 and 15°.

It is preferred that the light frame 100 comprises at least eight primary 131-134 and secondary 141-144 light sources, preferably at least twelve 131-138 primary and secondary 141-144 light sources, the latter example being illustrated in the Figures.

The light frame 100 preferably comprises a control means 160 arranged to control the flow of light from at least one or from a number of the light sources 131-138, 141-144. The control means 160 may be in the form of a dimmer switch means comprised in the light frame 100, by means of which the respective light output of the light sources 131-138, 141-144 can be adjusted, over a continuous range by a user, or be turned on or off.

According to a preferred embodiment, the primary 131-138 and secondary 141-144 light sources are divided into at least two groups of light sources, the light output of which can be controlled on a group level, by a user using the control means 160. In this case, it is preferred that at least one such group is a set of the above described pairs of primary or secondary light sources. For example, such a group may be comprised by a respective primary light source 131 on a first side, in the horizontal plane during use in said mounted state, of the sight glass 210, in combination with another respective primary light source 133 on the other, second, side sight glass 210, and so that the two light sources 131, 133 can be controlled in unison using the control means 160.

In the case in which the light sources 131-138, 141-144 cooperate in pairs in the manner described above, it is preferred that each such cooperating pair of light sources represents a respective group of light sources. Preferably, the light frame 100 comprises eight, more preferably twelve, light sources 131-138, 141-144, that are arranged in pairs in at least three, preferably four, preferably six groups, of two different light sources each, which groups are individually adjustable regarding light output using the above-described control means 160. In a preferred embodiment, a respective group comprises each primary light source which is directed towards one and the same single common point, and an additional group comprises all secondary light sources.

Moreover, it is preferred that the light frame 100 comprises a light sensor 170 for ambient brightness, in other words, a general lighting brightness that exists in connection to the light frame 100. In this case, the light frame 100 is arranged to read the ambient brightness using the light sensor 170 and to then automatically regulate down the flow of light from the primary 131-138 and/or secondary 141-144 light sources as a result of an increase in ambient brightness. For example, such a function may be used for automatic lighting control when welding with a welding helmet 200 on which the light frame 100 is mounted. It is preferred that at least the primary light sources 131-138 are subjected to such an automatic control, whereas the secondary light sources 141-144, in some embodiments, may be excepted from the said automatic down regulation.

Hence, according to a preferred embodiment, the light frame 100 is arranged to be attached to a visor, such as a visor being part of a welding helmet 200 (see Figures 2a and 2b).

In particular in the case of the use on a helmet with a visor, such as the said welding helmet 200, it is preferred that the light frame 100 comprises attachment means specifically adapted to engage with a certain geometric structure 210 of the helmet 200 and during such engagement to releasably retain the light frame 100 on the helmet 200 in the above mounted state, so that the light sources 131-138, 141-144 of the light frame 100 are arranged around a sight glass 210 of the helmet's 200 visor in the manner described above.

A preferred example of such attachment means are hook means, preferably a respective hook means 191 on either side of the visor sight glass 210, arranged to engage with a corresponding edge 220, or the like, of the visor.

As an alternative to such engaging fastening means, the light frame 100 can be arranged for use with a visor by being attached with a head-band 192 around the head of a user wearing the shield during normal use of the visor. This is illustrated in Figure 3.

It is preferred that the light frame 100 comprises a control device 180, which is connected to the light sources 131-138, 141-144, the voltage source 150, the control means 160 and the light sensor 170, and which is arranged to provide the functions described above. The control device 190 may be arranged with analogue and/or digital electronics for providing the said functions.

Above, a number of preferred embodiments have been described. It will be appreciated that many changes may be made to these embodiments without departing from the basic idea of the invention.

For example, each of the pairs of light sources described above may comprise more than two light sources, such as three, four or more light sources that cooperate as described above to together provide a good bright lighting of the working area and/or a good general lighting.

The light sources 131-138, 141-144 are preferably LED light sources, but, for example, halogen light sources are also conceivable.

Thus, the invention is not limited to the above described embodiments, but may be varied within the scope of the enclosed claims.

## Claims

1. Light frame (100) for use with a visor (200), which light frame comprises a rigid frame part (110) arranged to be mounted about the periphery of a sight glass (210) of the visor using fastening means (192,192) of the light frame (100) arranged for attaching the light frame (100) to the visor (200), which frame part comprises at least three directed light sources (131-138,141-144), with respective light cones (131a-138a,141a-144a), arranged to illuminate the area in front of sight glass during use, which light frame further comprises a voltage source (150) arranged to power the light sources, **characterised in** the combination that said light sources comprise at least two fixedly directed primary light sources (131-138), whose light cones (131a-138a) are directed towards one and the same point arranged between 20 and 80 cm in front of the sight glass during use, and **in that** said light sources further comprise at least one fixedly directed secondary light source (141-144), whose light cone (141a-144a) during use is not directed towards the said point, **in that** different pairs of respective primary light sources (131-138) are mounted on the frame part (110) so that they are disposed on different sides of the sight glass (210) during use, and **in that** at least two such pairs of primary light sources are arranged to, during use, be directed towards different common points (139a,139b) in front of the sight glass.

2. Light frame (100) according to claim 1, **characterised in that** the light frame furthermore comprises respective secondary light sources (141-144) mounted on the frame part so that they are disposed on either side of the sight glass during use.

3. Light frame (100) according to claim 1 or 2, **characterised in that** the light frame comprises at least two respective secondary light sources (141-144) mounted on the frame part (110) so that they are disposed on either side of the sight glass (210) during use, and so that these secondary light sources are disposed at a substantially horizontal distance from each other on the visor (200) during use, and **in that** this pair of secondary light sources are divergently directed with a relative angle between them of at the most 10°.

4. Light frame (100) according to claim 3, **characterised in that** the sight glass (210) has a substantially angular shape with outer corners, and **in that** respective secondary light sources (141-144) are mounted on the frame part (110) so that they are disposed at different outer corners of the sight glass during use, at three or more, preferably four, different locations.

5. Light frame (100) according to claim 3, **characterised in that** said different common points (139a,139b) in front of the sight glass are arranged at different respective distances from the sight glass for different such pairs.

6. Light frame (100) according to any one of the preceding claims, **characterise d in** that the light frame comprises at least a total of eight primary (131-138) and secondary (141-144) light sources.

7. Light frame (100) according to any one of the preceding claims, **characterised in that** the said primary (131-138) and secondary (141-144) light sources are divided into at least two different groups, the light output of which can be affected on a group level by a user using a control means (160) comprised in the light frame.

8. Light frame (100) according to any one of the preceding claims, **characterised in that** the light frame comprises a dimmer means (160) by the use of which the respective light output of the light sources (131-138,141-144) can be controlled across a continuous interval by a user.

9. Light frame (100) according to any one of the preceding claims, **characterised in that** the light frame comprises a light sensor (170) for ambient brightness, and **in that** the light frame is arranged to read the ambient brightness from the light sensor, and to automatically regulate down the light output from the said primary (131-138) and/or secondary (141-144) light sources as a result of an increased ambient brightness.

10. Light frame (100) according to any one of the preceding claims, **characterised in that** the fastening means are hook means (191), wherein the light frame is arranged to be fastened to a visor (200) using the e-spective hook means (191) on either side of the sight glass (210) of the visor.

11. Light frame (100) according to any one of the preceding claims, **characterised in that** the fastening means is a head-band (192), wherein the light frame is arranged for use with a visor by being fastened using the head-band (192) around the head of a user carrying the visor during normal use of the visor.

12. Light frame (100) according to any one of the preceding claims, **characterised in that** the visor (200) is constituted by or is a part of a welding helmet.

13. Welding helmet, comprising a sight glass and a light frame according to any of the preceding claims, whereby the light frame is arranged around the periphery of the sight glass.

## Patentansprüche

1. Lichtrahmen (100) zur Verwendung mit einem Visier (200), wobei der Lichtrahmen ein starres Rahmenteil (110) umfasst, das so angeordnet ist, dass es um den Umfang eines Sichtglases (210) des Visiers herum unter Verwendung von Befestigungsmitteln (192, 192) des Lichtrahmens (100), die so vorgesehen sind, dass sie den Lichtrahmen (100) an dem Visier (200) befestigen, montiert werden kann, wobei das Rahmenteil mindestens drei gerichtete Lichtquellen (131-138, 141-144) mit jeweiligen Lichtkegeln (131a-138a, 141a-144a), die so angeordnet sind, dass sie während des Gebrauchs den Bereich vor dem Sichtglas beleuchten, umfasst, wobei der Lichtrahmen ferner eine Spannungsquelle (150) umfasst, die so vorgesehen ist, dass sie die Lichtquellen versorgt, **gekennzeichnet durch** die Kombination, dass die Lichtquellen mindestens zwei fest ausgerichtete primäre Lichtquellen (131-138) umfassen, deren Lichtkegel (131a-138a) während des Gebrauchs auf ein und denselben Punkt gerichtet sind, der zwischen 20 und 80 cm vor dem Sichtglas angeordnet ist, und dass die Lichtquellen ferner mindestens eine fest ausgerichtete sekundäre Lichtquelle (141-144) umfassen, deren Lichtkegel (141a-144a) während des Gebrauchs nicht auf den genannten Punkt gerichtet ist, dass verschiedene Paare von jeweiligen primären Lichtquellen (131-138) an dem Rahmenteil (110) so angebracht sind, dass sie während des Gebrauchs auf verschiedenen Seiten des Sichtglases (210) angeordnet sind, und dass mindestens zwei solcher Paare von primären Lichtquellen so angeordnet sind, dass sie während des Gebrauchs auf verschiedene gemeinsame Punkte (139a, 139b) vor dem Sichtglas gerichtet sind.

2. Lichtrahmen (100) nach Anspruch 1, **dadurch gekennzeichnet, dass** der Lichtrahmen jeweilige sekundäre Lichtquellen (141-144) umfasst, die auf dem Rahmenteil so montiert sind, dass sie während des Gebrauchs auf beiden Seiten des Sichtglases angeordnet sind.

3. Lichtrahmen (100) nach Anspruch 1 oder 2, **dadurch gekennzeichnet, dass** der Lichtrahmen mindestens zwei jeweilige sekundäre Lichtquellen (141-144) umfasst, die auf dem Rahmenteil (110) so montiert sind, dass sie während des Gebrauchs auf beiden Seiten des Sichtglases (210) angeordnet sind, und dass diese sekundären Lichtquellen während des Gebrauchs in einem im Wesentlichen horizontalen Abstand voneinander auf dem Visier (200) angeordnet sind, und dass dieses Paar sekundärer Lichtquellen mit einem relativen Winkel von höchstens 10° zwischen ihnen divergent ausgerichtet sind.

4. Lichtrahmen (100) nach Anspruch 3, **dadurch gekennzeichnet, dass** das Sichtglas (210) eine im Wesentlichen eckige Form mit Außenecken aufweist, und dass jeweilige sekundäre Lichtquellen (141-144) auf dem Rahmenteil (110) so montiert sind, dass sie während des Gebrauchs an verschiedenen Außenecken des Sichtglases an drei oder mehr, vorzugsweise vier verschiedenen Stellen angeordnet sind.

5. Lichtrahmen (100) nach Anspruch 3, **dadurch gekennzeichnet, dass** die verschiedenen gemeinsamen Punkte (139a, 139b) vor dem Sichtglas für verschiedene solcher Paare in unterschiedlichen jeweiligen Abständen vom Sichtglas angeordnet sind.

6. Lichtrahmen (100) nach einem der vorstehenden Ansprüche, **dadurch gekennzeichnet, dass** der Lichtrahmen insgesamt mindestens acht primäre (131-138) und sekundäre (141-144) Lichtquellen umfasst.

7. Lichtrahmen (100) nach einem der vorstehenden Ansprüche, **dadurch gekennzeichnet, dass** die primären (131-138) und sekundären (141-144) Lichtquellen in mindestens zwei verschiedene Gruppen unterteilt sind, deren Lichtleistung auf Gruppenebene von einem Benutzer mit Hilfe eines im Lichtrahmen enthaltenen Steuermittels (160) beeinflusst werden kann.

8. Lichtrahmen (100) nach einem der vorstehenden Ansprüche, **dadurch gekennzeichnet, dass** der Lichtrahmen eine Dimmeinrichtung (160) umfasst, durch deren Verwendung die jeweilige Lichtleistung der Lichtquellen (131-138, 141-144) über einen kontinuierlichen Bereich hinweg von einem Benutzer gesteuert werden kann.

9. Lichtrahmen (100) nach einem der vorstehenden Ansprüche, **dadurch gekennzeichnet, dass** der Lichtrahmen einen Lichtsensor (170) für die Umgebungshelligkeit umfasst, und dass der Lichtrahmen so vorgesehen ist, dass er die Umgebungshelligkeit vom Lichtsensor ausliest und die Lichtleistung von den primären (131-138) und/oder sekundären (141-144) Lichtquellen als Ergebnis einer erhöhten Umgebungshelligkeit automatisch herunterreguliert.

10. Lichtrahmen (100) nach einem der vorstehenden Ansprüche, **dadurch gekennzeichnet, dass** die Befestigungsmittel Hakenmittel sind, wobei der Lichtrahmen so vorgesehen ist, dass er an einem Visier (200) unter Verwendung der jeweiligen Hakenmittel (191) auf beiden Seiten des Sichtglases (210) des Visiers befestigt werden kann.

11. Lichtrahmen (100) nach einem der vorstehenden Ansprüche, **dadurch gekennzeichnet, dass** das Befestigungsmittel ein Stirnband ist, wobei der Lichtrahmen zur Verwendung mit einem Visier vorgesehen ist, indem er unter Verwendung des Stirnbandes (192) um den Kopf eines Benutzers, der während des normalen Gebrauchs des Visiers das Visier trägt, befestigt wird.

12. Lichtrahmen (100) nach einem der vorstehenden Ansprüche, **dadurch gekennzeichnet, dass** das Visier (200) aus einem Schweißhelm besteht oder ein Teil eines Schweißhelms ist.

13. Schweißhelm, umfassend ein Sichtglas und einen Lichtrahmen nach einem der vorstehenden Ansprüche, wobei der Lichtrahmen um den Umfang des Sichtglases herum angeordnet ist.

## Revendications

1. Cadre lumineux (100) destiné à être utilisé avec une visière (200), lequel cadre lumineux comprend une partie de cadre rigide (110) prévue pour être montée autour de la périphérie d'un verre d'observation (210) de la visière à l'aide d'un moyen de fixation (192, 192) du cadre lumineux (100) prévu pour fixer le cadre lumineux (100) à la visière (200), laquelle partie de cadre comprend au moins trois sources de lumière orientée (131-138, 141-144), avec des cônes de lumière respectifs (131a-138, 141a-144a), prévues pour éclairer la zone située en face du verre d'observation pendant l'utilisation, lequel cadre lumineux comprend en outre une source de tension (150) prévue pour alimenter les sources de lumière, **caractérisé en ce que** lesdites sources de lumière comprennent au moins deux sources de lumière principales orientées de manière fixe (131-138), dont les cônes de lumière (131a-138a) sont orientés vers un seul et même point prévu entre 20 et 80 cm en face du verre d'observation pendant l'utilisation, et **en ce que** lesdites sources de lumière comprennent en outre au moins une source de lumière secondaire orientée de manière fixe (141-144), dont le cône de lumière (141a-144a) pendant l'utilisation n'est pas orienté vers ledit point, **en ce que** différentes paires de sources de lumière principales respectives (131-138) sont montées sur la partie de cadre (110) de façon à être disposées sur différents côtés du verre d'observation (210) pendant l'utilisation, et **en ce que** au moins deux de ces paires de sources de lumière principales sont prévues, pendant l'utilisation, pour être orientées vers différents points communs (139a, 139b) en face du verre d'observation.

2. Cadre lumineux (100) selon la revendication 1, **caractérisé en ce que** le cadre lumineux comprend en outre des sources de lumière secondaires respectives (141-144) montées sur la partie de cadre de façon à être disposées de n'importe quel côté du verre d'observation pendant l'utilisation.

3. Cadre lumineux (100) selon la revendication 1 ou 2, **caractérisé en ce que** le cadre lumineux comprend au moins deux sources de lumière secondaires respectives (141-144) montées sur la partie de cadre (110) de façon à être disposées de n'importe quel côté du verre d'observation (210) pendant l'utilisation, et de sorte que ces sources de lumière secondaires soient disposées à une distance sensiblement horizontale les unes des autres sur la visière (200) pendant l'utilisation, et **en ce que** cette paire de sources de lumière secondaires est orientée de manière divergente avec un angle relatif entre celles-ci de 10° tout au plus.

4. Cadre lumineux (100) selon la revendication 3, **caractérisé en ce que** le verre d'observation (210) possède une forme sensiblement angulaire avec des angles externes, et **en ce que** les sources de lumière secondaires respectives (141-144) sont montées sur la partie de cadre (110) de façon à être disposées au niveau de différents angles externes du verre d'observation pendant l'utilisation, à trois ou plus, de préférence quatre, emplacements différents.

5. Cadre lumineux (100) selon la revendication 3, **caractérisé en ce que** lesdits différents points communs (139a, 139b) en face du verre d'observation sont prévus à différentes distances respectives du verre d'observation pour différentes paires de ce type.

6. Cadre lumineux (100) selon l'une quelconque des revendications précédentes, **caractérisé en ce que** le cadre lumineux comprend au moins un total de huit sources de lumière principales (131-138) et secondaires (141-144).

7. Cadre lumineux (100) selon l'une quelconque des revendications précédentes, **caractérisé en ce que** lesdites sources de lumière principales (131-138) et secondaires (141-144) sont divisées en au moins deux groupes différents, dont le rendement lumineux peut être affecté au niveau d'un groupe par un utilisateur qui se sert d'un moyen de commande (160) compris dans le cadre lumineux.

8. Cadre lumineux (100) selon l'une quelconque des revendications précédentes, **caractérisé en ce que** le cadre lumineux comprend un moyen de variation d'intensité (160) à l'aide duquel le rendement lumineux respectif des sources de lumière (131-138, 141-144) peut être contrôlé sur un intervalle continu par un utilisateur.

9. Cadre lumineux (100) selon l'une quelconque des revendications précédentes, **caractérisé en ce que** le cadre lumineux comprend un capteur de lumière (170) destiné à la luminosité ambiante, et **en ce que** le cadre lumineux est prévu pour lire la luminosité ambiante indiquée par le capteur de lumière, et pour réguler automatiquement à la baisse le rendement lumineux desdites sources de lumière principales (131-138) et/ou secondaires (141-144) suite à une augmentation de la luminosité ambiante.

10. Cadre lumineux (100) selon l'une quelconque des revendications précédentes, **caractérisé en ce que** le moyen de fixation est un crochet (191), dans lequel le cadre lumineux est prévu pour être fixé à une visière (200) à l'aide du crochet respectif (191) de n'importe quel côté du verre d'observation (210) de la visière.

11. Cadre lumineux (100) selon l'une quelconque des revendications précédentes, **caractérisé en ce que** le moyen de fixation est un bandeau élastique (192), dans lequel le cadre lumineux est prévu pour être utilisé avec une visière en étant fixé à l'aide du bandeau élastique (192) autour de la tête d'un utilisateur qui porte la visière pendant l'utilisation normale de la visière.

12. Cadre lumineux (100) selon l'une quelconque des revendications précédentes, **caractérisé en ce que** la visière (200) est constituée de ou fait partie d'un casque de soudeur.

13. Casque de soudeur, comprenant un verre d'observation et un cadre lumineux selon l'une quelconque des revendications précédentes, moyennant quoi le cadre lumineux est prévu autour de la périphérie du verre d'observation.
